# EUROPÄISCHE PATENTSCHRIFT

(11) **EP 2 551 698 B1**
(45) Veröffentlichungstag und Bekanntmachung des Hinweises auf die Patenterteilung: **17.09.2014**
(21) Anmeldenummer: 11006252.8
(22) Anmeldetag: 29.07.2011
(51) Int. Cl.: G01S 17/36, A61B 1/04, A61B 5/06, A61B 19/00, A61B 1/00, A61B 5/00

(54) **Endoskopisches Instrument**
Endoscopic instrument
Instrument endoscopique

(43) Veröffentlichungstag der Anmeldung: 30.01.2013
(73) Patentinhaber: Richard Wolf GmbH, 75438 Knittlingen (DE)
(72) Erfinder: Heckele, Helmut, 75438 Knittlingen (DE); Völlinger, Hubert, 76437 Rastatt (DE)
(74) Vertreter: Patentanwälte Vollmann & Hemmer

(56) Entgegenhaltungen:
- EP-A1- 1 952 752
- EP-A2- 2 026 034
- WO-A1-2005/077272
- DE-A1- 10 323 217

## Beschreibung

Die Erfindung betrifft ein endoskopisches Instrument.

Bei einer Intervention mit endoskopischen Instrumenten in tiefen, von außen nicht einsehbaren Regionen des Körpers ist die Visualisierung des Operationsgebietes von großer Bedeutung. Endoskope sind dabei eine wesentliche Hilfe. Systembedingt finden Endoskope aber dort ihre Grenzen, wo es unter Risikoerwägungen heraus nötig ist, hinter dem augenblicklich betrachteten oberflächlichen Bild verborgene Strukturen zu sehen und bei der Operation zu berücksichtigen. Es ist daher wünschenswert, Objekte bzw. Bereiche des Operationsgebietes digital zu rekonstruieren, um auch aktuell nicht einsehbare Bereiche berücksichtigen zu können.

WO 2005/077272 A1 offenbart ein Verfahren zum Erstellen zumindest eines Ausschnittes eines virtuellen 3D-Modells eines Körperinnenraumes. Hierzu ist ein endoskopisches Instrument vorgesehen, welches ein Trägheitssensorsystem sowie ein Abstandsmesssystem, welches beispielsweise mit Hilfe eines Ultraschallsignals oder eines Laserstrahls den Abstand einzelner Punkte in einem aufgenommenen Bild von dem Instrument bestimmt.

EP 2 026 034 A2 offenbart eine Vorrichtung zur Ermittlung der 3D-Koordinaten eines Objektes, insbesondere eines Zahns. Hierzu wird ein mit Lagesensoren versehenes Scansystem verwendet, welches die Zahnoberfläche abscannt.

Es ist daher Aufgabe der Erfindung ein endoskopisches Instrument dahingehend zu verbessern, dass eine umfassende Visualisierung bzw. Rekonstruktion eines zu untersuchenden bzw. zu operierenden Objektes möglich wird.

Diese Aufgabe wird durch ein endoskopisches Instrument mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße endoskopische Instrument weist zumindest einen optischen Sensor und zumindest einen Lagesensor auf. Dabei ist der Lagesensor dazu geeignet, eine Lageänderung des Blickfeldes des optischen Sensors im Raum zu erfassen. D. h. über den Lagesensor kann insbesondere die Blickrichtung des optischen Sensors erfasst werden. Erfindungsgemäß weist das endoskopische Instrument darüber hinaus eine Bildverarbeitungseinrichtung auf, welche so ausgestaltet ist, dass sie die Ausgangssignale des optischen Sensors und Ausgangssignale des Lagesensors gemeinsam verarbeitet. D. h. bei der Bildverarbeitung kann in der Bildverarbeitungseinrichtung die Blickrichtung des optischen Sensors berücksichtigt werden. Dazu ist die Bildverarbeitungseinrichtung so ausgestaltet, dass sie von dem optischen Sensor in die verschiedenen Lagen des Blickfeldes, d. h. in verschiedenen Blickrichtungen erfasste Bildinformationen unter Berücksichtigung der Lage des Blickfeldes zu einem Gesamtbild eines betrachteten Objektes zusammensetzt. D. h. es können verschiedene Ansichten aus verschiedenen Blickrichtungen, welche von dem optischen Sensor erfasst werden, in der Bildverarbeitungseinrichtung so miteinander kombiniert werden, dass ein umfassendes Gesamtbild des Objektes erzeugt wird. Dieses Gesamtbild kann dann beispielsweise Bildinformationen enthalten, welche aus einzelnen Blickrichtungen nicht erkennbar sind, jedoch aus anderen Blickrichtungen. So kann ein Gesamtbild bzw. Modell des betrachteten Objektes der Bildbearbeitungseinrichtung erzeugt werden, welches dann bei der weiteren Diagnose oder Operation berücksichtigt werden kann.

Diese Bildverarbeitungseinrichtung ist vorzugsweise in ein Computersystem integriert, welches mit dem eigentlichen Endoskop, d. h. dem Instrument, welches in eine Körperöffnung eingeführt wird, über eine geeignete Datenverbindung, beispielsweise ein Datenkabel verbunden ist. Die Bildverarbeitungseinrichtung muss somit nicht direkt in das eigentliche Endoskop integriert werden, sondern stellt einen Bestandteil des endoskopischen Instrumentes bzw. des endoskopischen Instrumentensystems dar, welches von dem eigentlichen Endoskop beabstandet angeordnet werden kann. Die Bildverarbeitungsvorrichtung ist vorzugsweise mit zumindest einem Monitor verbunden, welcher eine Betrachtung des Objektes ermöglicht. Dabei kann es möglich sein, das Objekt am Monitor zu drehen bzw. virtuell aus verschiedenen Blickrichtungen zu betrachten, in denen zuvor von dem optischen Sensor Bildinformationen aufgenommen wurden.

Die Bildverarbeitungseinrichtung ist derart ausgestaltet, dass sie Ausgangsignale des optischen Sensors und Ausgangssignale des Lagesensors gemeinsam derart verarbeitet, dass von dem optischen Sensor in verschiedenen Lagen des Blickfeldes erfasste Bildinformationen unter Berücksichtigung der Lage des Blickfeldes zu einem dreidimensionalen Gesamtbild des betrachteten Objektes zusammengesetzt werden. D. h. vorzugsweise wird ein dreidimensionales Modell des Objektes erzeugt, welches eine Betrachtung von verschiedenen Seiten ermöglicht, sodass auch Hinterschneidungen und ähnliches betrachtet werden können.

Der optische Sensor ist weiter bevorzugt ein Videosensor, welcher vorzugsweise am distalen Ende des Endoskops angeordnet ist. Vom distalen Ende können die Bildinformationen über geeignete Datenleitungen, gegebenenfalls über Funk zu der Bildverarbeitungseinrichtung übertragen werden. Alternativ ist es auch möglich, den optischen Sensor am proximalen Ende des Endoskops anzuordnen und ein entsprechendes optisches System zur Bildübertragung vom distalen Ende zum proximalen Ende vorzusehen.

Der Lagesensor weist bevorzugt zumindest zwei Bewegungsaufnehmer auf, über welche Lageänderungen im Raum erfassbar sind. Diese Bewegungsaufnehmer sind sehr klein und können leicht in das Instrument bzw. das Endoskop integriert werden, so dass auf umständlich zu handhabende, bei der Operation störende Marker verzichtet werden kann, um die Lage des Instrumentes im Raum zu erfassen. Die Bewegungsaufnehmer geben Ausgangssignale ab, welche einer Auswerteeinrichtung zugeführt werden, welche Bestandteil des endoskopischen Instrumentes ist und in dieses integriert oder als separate Baueinheit mit diesem, beispielsweise über einen Datenbus, verbunden sein kann. Die Auswerteeinrichtung ist so ausgebildet, dass sie auf Grundlage der Ausgangssignale der Bewegungsaufnehmer Veränderungen der Lage des Endoskops und der Lage des Blickfeldes des optischen Sensors im Raum bestimmen kann. Hierzu können mehrere Bewegungsaufnehmer vorgesehen sein, welche Bewegungen in unterschiedlichen Raumrichtungen erfassen, vorzugsweise in allen erforderlichen sechs Freiheitsgraden. Diese Auswerteeinrichtung des Lagesensors kann in die Bildverarbeitungseinrichtung integriert sein oder mit dieser auf einem gemeinsamen Computersystem ausgeführt werden.

Bevorzugt ist zumindest einer der Bewegungsaufnehmer ein Beschleunigungsaufnehmer, d. h. ein Sensor, welcher Beschleunigungen entlang einer oder mehrerer Achsen erfassen kann.

Gemäß einer weiteren Ausführungsform kann zumindest einer der Bewegungsaufnehmer ein Drehratensensor sein, durch welchen die Rotationsgeschwindigkeit und Winkellagen-Änderungen bezüglich einer oder mehrerer Achsen erfasst werden können. Bevorzugt wird bei dieser Ausführungsform zumindest ein Beschleunigungssensor und ein Drehratenaufnehmer in Kombination eingesetzt, um Lageänderungen des Instrumentes im Raum zu erfassen.

Weiter ist es bevorzugt, dass die zumindest zwei Bewegungsaufnehmer in dem Instrument beabstandet zueinander angeordnet sind. Auf diese Weise wird die Lageerfassung im Raum verbessert bzw. eine genauere Positionserfassung möglich.

Vorzugsweise ist zumindest einer der Bewegungsaufnehmer als ein Mehr-Achsen-Beschleunigungsaufnehmer, z. B. als Drei-Achsen-Beschleunigungsaufnehmer ausgebildet. Das heißt, dieser Beschleunigungsaufnehmer kann Beschleunigungen in mehreren, vorzugsweise drei zueinander orthogonalen, Richtungen erfassen und als entsprechende Ausgangssignale ausgeben. Weiter bevorzugt sind zumindest zwei derartige Mehr-Achsen-Beschleunigungsaufnehmer in dem Instrument angeordnet. Durch Kombination bzw. gemeinsame Auswertung der Ausgangssignale von zwei Drei-Achsen-Beschleunigungsaufnehmern ist es möglich, neben den Beschleunigungen bzw. Bewegungen entlang der Achsen der Beschleunigungsaufnehmer auch noch Dreh- bzw. Schwenkbewegungen des Instrumentes um diese Achsen zu erfassen, so dass alle sechs Freiheitsgrade erfassbar sind. Auch ist die Kombination eines oder mehrerer Mehr-Achsen-Beschleunigungsaufnehmer und zumindest eines Drehratenaufnehmers denkbar, um Schwenkbewegungen des Instrumentes zu erfassen. Anstelle von Mehr-Achsen-Beschleunigungsaufnehmern können auch mehrere einachsige Beschleunigungsaufnehmer Verwendung finden. Die Auswertung der Ausgangssignale der Aufnehmer erfolgt in der entsprechend ausgebildeten Auswerteeinrichtung.

Vorzugsweise ist zumindest einer der Bewegungsaufnehmer in der distalseitigen Hälfte, insbesondere im distalen Bereich des Instrumentes angeordnet. Weiter bevorzugt ist zumindest ein Bewegungsaufnehmer im Bereich des distalen Endes des Instrumentes, d. h. vorzugsweise in der Nähe der Instrumentenspitze, angeordnet. Durch die Lage des Aufnehmers bzw. Sensors an dieser Position wird insbesondere die genaue Ermittlung der Lage bzw. Verfolgung der Bewegung der Spitze des Instrumentes und des Blickfeldes des optischen Sensors im Raum möglich.

Gemäß einer weiteren bevorzugten Ausführungsform ist zumindest ein Bewegungsaufnehmer in der proximalseitigen Hälfte, insbesondere im proximalen Bereich des Instrumentes angeordnet. Grundsätzlich ist es zur Erfassung der Lage im Raum in allen sechs Freiheitsgraden durch Verwendung mehrerer Beschleunigungsaufnehmer, insbesondere bei Verwendung von zumindest zwei Mehr-Achsen-Beschleunigungsaufnehmern erforderlich, zwei der Beschleunigungsaufnehmer beabstandet zueinander anzuordnen, um durch unterschiedliche Beschleunigungswerte an den beiden Beschleunigungsaufnehmern Schwenkbewegungen des medizinischen Instrumentes erfassen zu können. Ein maximaler Abstand kann erreicht werden, wenn einer der Bewegungsaufnehmer am distalen Ende bzw. im distalen Bereich und ein anderer Bewegungsaufnehmer am proximalen Ende bzw. im proximalen Bereich des Instrumentes angeordnet ist.

Ferner ist es bevorzugt, zumindest einen der Bewegungsaufnehmer in einer am proximalen Ende des Instrumentes ausgebildeten Handhabe anzuordnen. Hierdurch ist zum einen eine möglichst nahe dem proximalen Ende gelegene Anordnung des Bewegungsaufnehmers möglich, zum anderen ist in der Handhabe ausreichend Raum zur Anordnung eines oder mehrerer Bewegungsaufnehmer vorhanden. So können auch zwei Bewegungsaufnehmer, vorzugsweise beabstandet zueinander, in der Handhabe angeordnet sein. So können beispielsweise ein erster Bewegungsaufnehmer im Bereich des distalen Endes der Handhabe und ein zweiter Bewegungssaufnehmer im Bereich des proximalen Endes der Handhabe angeordnet sein.

Gemäß einer weiteren bevorzugten Ausführungsform können zumindest zwei Bewegungsaufnehmer an diametral entgegengesetzten Seiten der Instrumentenlängsachse, d. h. der sich vom proximalen zum distalen Ende erstreckenden Achse angeordnet sein. Auch hierdurch kann eine beabstandete Anordnung der Bewegungsaufnehmer geschaffen werden. Ferner ist dies vorteilhaft, um Drehungen um die Instrumentenlängsachse zu erfassen.

Gemäß einer weiteren bevorzugten Ausführungsform weist das endoskopische Instrument einen Schaft mit zumindest einem abwinkelbaren oder biegbaren Schaftabschnitt auf, wobei zumindest ein Bewegungs-, insbesondere Beschleunigungsaufnehmer in dem abwinkelbaren Schaftabschnitt angeordnet ist. Dies ermöglicht es, über diesen Bewegungsaufnehmer die Bewegung beim Abwinkeln bzw. Biegen des Schaftabschnittes zu erfassen und damit durch die Biegung bzw. Auslenkung verbundene Blickrichtungsänderungen zu erfassen. D. h. insbesondere dann, wenn der optische Sensor bzw. ein Fenster eines optischen Systems, welches mit dem optischen Sensor in Verbindung steht, am distalen Ende des Instrumentes in einem biegbaren Schaftabschnitt angeordnet ist, ist es hilfreich, die genaue Auslenkung bzw. Ausrichtung des biegbaren Schaftabschnittes im Raum zu erfassen, da damit dann auch die Blickrichtung des optischen Sensors, d. h. die Lage des Blickfeldes des optischen Sensors im Raum erfasst werden kann. Gegebenenfalls können die Lageinformationen, welche der Beschleunigungsaufnehmer in dem abwinkelbaren oder biegbaren Schaftabschnitt liefert, mit den Lageinformationen weiterer Bewegungsaufnehmer in anderen Teilen des Instrumentes kombiniert bzw. gemeinsam ausgewertet werden, um die Lage des Blickfeldes im Raum zu bestimmen.

Ferner ist es bevorzugt, dass die Auswerteeinrichtung des Lagesensors derart ausgestaltet ist, dass sie eine Kalibrierfunktion aufweist, mittels welcher der Auswerteeinrichtung eine definierte Ausgangslage des Instrumentes eingebbar ist, von der ausgehend von der Auswerteeinrichtung Veränderungen der Lage des Instrumentes bzw. insbesondere dessen distalen Endes und Blickfeldes des optischen Sensors im Raum bestimmbar sind. Über die Bewegungs-, insbesondere Beschleunigungsaufnehmer sind nur Bewegungen des Instrumentes in verschiedenen Richtungen, insbesondere in allen sechs Freiheitsgraden, erfassbar. Um die Lage des Instrumentes während der Operation mit vorher erfassten Patientendaten abgleichen zu können, ist es jedoch erforderlich, stets die absolute Position des Instrumentes zu erfassen. Hierzu kann das Instrument zunächst in eine definierte Ausgangslage, vorzugsweise in eine definierte Ausgangslage relativ zu dem Patienten gebracht werden und diese Ausgangslage dann über die Kalibrierfunktion der Auswerteeinrichtung eingegeben werden. Ausgehend von der Ausgangslage kann dann die Auswerteeinrichtung auf Grundlage der Ausgangssignale der Bewegungsaufnehmer Bewegungen des Instrumentes im Raum erfassen und somit aus den Bewegungen auf Grundlage der definierten Ausgangslage auf die neue absolute Lage im Raum schließen. So kann stets die aktuelle Lage des Instrumentes vorzugsweise in Echtzeit von der Auswerteeinrichtung erfasst werden.

Erfindungsgemäß ist der optische Sensor Teil eines TOF-Kamerasystems, d. h. eines Time-Of-Flight-Kamerasystems. Derartige Kamerasysteme sind beispielsweise aus DE 44 40 613 C1, EP 1 952 752 oder EP 1 622 200 A1 bekannt. Bei TOF-Kameras handelt es sich um 3D-Kamerasysteme, welche mittels des Laufzeitverfahrens Distanzen messen. D. h. auf Grundlage der Laufzeit der Lichtsignale von einzelnen Bildpunkten kann der Abstand dieser Bildpunkte vom Bildsensor, d. h. dem optischen Sensor bestimmt werden. Auf diese Weise kann ein dreidimensionales Bild erzeugt werden. So kann durch die Kombination eines solchen Kamerasystems mit dem oben beschriebenen Lagesensor eine genauere Objektrekonstruktionen erreicht werden, d. h. ein genaueres dreidimensionales Abbild des zu untersuchenden Objektes von der Bildverarbeitungseinrichtung geschaffen werden. Unter Zuhilfenahme des so rekonstruieren Objektes können dann weitere Diagnosen und Operationen durchgeführt werden.

Der optische Sensor, welcher Teil eines TOF-Kamerasystems ist, stellt den Bildaufnehmer dieses Kamerasystems dar. Zusätzlich werden die üblichen Komponenten eines solchen TOF-Kamerasystems in das endoskopische Instrument bzw. das endoskopische Instrumentensystem integriert, d. h. insbesondere eine Auswerteeinrichtung und eine geeignete Beleuchtungseinrichtung. Die Auswerteeinrichtung kann dabei in die Bildverarbeitungseinrichtung, wie sie oben beschrieben wurde, integriert sein. D. h. insbesondere kann die Auswerteeinrichtung in ein Computersystem integriert sein, welche Teil des endoskopischen Instrumentensystems ist und mit dem eigentlichen Endoskop bzw. Instrument über eine geeignete Datenverbindung verbunden ist.

Das TOF-Kamerasystem weist eine Strahlenquelle auf, welche eine modulierte Strahlung aussendet. Diese Strahlenquelle ist in dem Instrument derart angeordnet, dass ein zu untersuchendes Objekt mit der Strahlung beleuchtbar ist. Dazu ist die Strahlenquelle entweder am distalen Ende des Endoskops angeordnet oder jedoch weiter proximalwärts oder außerhalb des Endoskops, wobei in letzteren Fällen dann ein Wellenleiter zum Leiten der Strahlung zum distalen Ende des Endoskops hin vorgesehen sein muss. Auf jeden Fall tritt die Strahlung am distalen Ende des Endoskops aus und bestrahlt das zu untersuchende Objekt bzw. einen Bereich des zu untersuchenden Objektes.

Der optische Sensor weist mehrere Sensorelemente auf. Der optische Sensor bzw. die Sensorelemente dienen zum Erfassen der modulierten Strahlung, welche von dem Objekt reflektiert wird. Dabei ist, insbesondere durch mehrere Sensorelemente, vorgesehen, die reflektierte Strahlung an verschiedenen Bildpunkten zu erfassen. Diese entsprechen verschiedenen Punkten des Objektes. Der optische Sensor mit den mehreren Sensorelementen bildet einen Flächensensor, wobei die einzelnen Sensorelemente die Strahlung einzelner Bildpunkte erfassen. Vorzugsweise ist der optische Sensor so ausgebildet, dass eine Signalerfassung an mehreren oder allen Sensorelementen gleichzeitig erfolgen kann, sodass ein vollständiges Bild von diesem Flächensensor aufgenommen werden kann. D. h. es gibt nicht ein einzelnes Sensorelement, welches das Bild abscannt, vielmehr werden mehrere Bildpunkte gleichzeitig durch mehrere Sensorelemente aufgenommen. Die einzelnen Sensorelemente können Speicherzellen zum Speichern der Bildinformationen aufweisen, um diese später auslesen zu können.

Ferner bildet die Bildverarbeitungseinrichtung eine Auswerteeinrichtung, welche die von dem Sensor aufgrund der erfassten reflektierten Strahlung generierten Messwerte erhält und basierend auf diesen Messwerten die Entfernung des Objektes von dem optischen Sensor errechnet. Dabei werden insbesondere einzelnen Bildpunkten entsprechende Punkte des Objektes vermessen, d. h. die Entfernung dieser Punkte von dem Sensor bestimmt. Die Entfernungsmessung erfolgt in der Bildverarbeitungs- bzw. Auswerteeinrichtung derart, dass insbesondere in Kenntnis der Modulation der Strahlenquelle aus den Messwerten an den einzelnen Sensorelementen Phasendifferenzen in der Strahlung ermittelt werden. Aus diesen kann dann auf die Signallaufzeit zwischen der Strahlenquelle und den einzelnen Sensorelementen geschlossen werden. Aus dieser Laufzeit kann dann die Entfernung der den einzelnen Sensorelementen zugeordneten Bildpunkte bestimmt werden.

Die Bildbearbeitungseinrichtung ermöglicht es durch die Entfernungsmessung ein zu beobachtendes bzw. zu untersuchendes Objekt in seiner dreidimensionalen Ausdehnung zu vermessen. Hier wird bevorzugt eine bestimmte Anzahl von Bildpunkten erfasst, sodass ein dreidimensionales Abbild des Objektes erzeugt werden kann. Dieses dreidimensionale Abbild kann dann mit den Informationen des zumindestens einen Lagesensors so kombiniert werden, dass auch die Lage des Blickfeldes bzw. die Blickrichtung des optischen Sensors bei der Erzeugung des dreidimensionalen Bildes von der Bildverarbeitungseinrichtung mitberücksichtigt wird.

Die Beleuchtung von visuell zu betrachtenden und darzustellenden dreidimensionalen Strukturen erfolgt vorzugsweise mittels einer Strahlen- bzw. Lichtquelle, die moduliertes Licht bevorzugt im sichtbaren Bereich aussendet. Dies ermöglicht es, die Optiken bzw. optischen Systeme und Lichtleiter von herkömmlichen Endoskopen zur Übertragung der modulierten Strahlung zu nutzen. So wird es insbesondere ermöglicht, ein TOF-Kamerasystem zur dreidimensionalen Erfassung von Objekten in herkömmliche Endoskope zu integrieren bzw. bei herkömmlichen Endoskopen nachzurüsten.

Diese modulierte Strahlenquelle ist weiter bevorzugt zum Aussenden von gepulstem Licht ausgebildet. D. h. das von der Strahlenquelle ausgesendete Licht wird in einem vorgegebenen Takt gepulst. Das modulierte Licht kann dadurch beispielsweise für jene Zeitspannen, in welchen ein Videosensor zur visuellen Betrachtung genutzt wird, inaktiv geschaltet werden und somit unerwünschten Einflüssen auf die Farbwahrnehmung des Systems entgegenwirken.

Vorzugsweise ist dazu ein Taktgenerator zur Steuerung der Strahlenquelle und des optischen Sensors und der Bildverarbeintungseinrichtung vorgesehen. Der Taktgenerator gibt zum einen die Modulation und die Pulsfrequenz des ausgesendeten Lichtes vor. Zum anderen gibt der Taktgeber auch den Takt für die Erfassung der reflektierten Strahlung an dem Sensor und die nachfolgende Auswertung vor. So ist es zur Erfassung von Phasendifferenzen für die Bildverarbeitungseinrichtung erforderlich, den ursprünglichen Phasenverlauf der von der Strahlenquelle ausgesandten Strahlung zu kennen, um dann aus dem Messergebnis des Sensors bzw. der Sensorelemente die Phasendifferenzen bestimmen zu können.

Gemäß einer bevorzugten Ausführungsform ist ein erster optischer Sensor zum Erfassen der von einem Objekt reflektierten Strahlung und ein Videosensor oder eine Optik mit einem Okular zur direkten Betrachtung des Objektes vorhanden. D. h. der erste optische Sensor ist Teil des TOF-Kamerasystems, welches die Entfernungsmessung und Errechnung eines dreidimensionalen Abbildes ermöglicht, während der Videosensor oder eine Optik mit dem Okular zur direkten Betrachtung des Objektes dienen. Besonders bevorzugt kann der optische Sensor, welcher Bestandteil des TOF-Systems ist, direkt in den Videosensor integriert sein, d. h. mit dem Videosensor eine bauliche Einheit bilden, oder der Videosensor selber kann die erforderliche TOF-Funktionalität in Verbindung mit einer zugehörigen Auswerteeinrichtung, d. h. des entsprechend angepassten Bildverarbeitungseinrichtung bereitstellen.

Für den Fall, dass ein optischer Sensor für das TOF-System und ein zusätzlicher Bildsensor für ein Okular zur direkten Bildbetrachtung vorhanden sein sollten, ist es möglich, dass der erste optische Sensor einerseits und der Videosensor oder das Okular andererseits einen gemeinsamen optischen Pfad nutzen. Der optische Pfad kann mit einem Sichtfenster am distalen Ende des endoskopischen Instrumentes verbunden sein, während das Okular oder der Videosensor und der optische Sensor des TOF-Systems weiter proximalwärts, vorzugsweise am proximalen Ende des Endoskops angeordnet werden können. Durch Verwendung des gemeinsamen optischen Pfades wird Bauraum im Schaft des Endoskops gespart, da nur ein einziges Bildübertragungssytem erforderlich ist.

Das TOF-Kamerasystem lässt sich auf verschiedene Weise in ein endoskopisches Instrument integrieren. So ist es möglich, die Strahlenquelle sowie den Sensor zum Erfassen der reflektierten Strahlung am proximalen Ende des Endoskops oder an das proximale Ende angeschlossen anzuordnen und die modulierte Strahlung zum distalen Ende und die reflektierte Strahlung zum proximalen Ende über Lichtleiter bzw. optische Systeme im Endoskop zu leiten. Alternativ ist es möglich, die Strahlenquelle und/oder den optischen Sensor direkt am distalen Ende des Endoskops oder im Inneren des Endoskops zwischen proximalem und distalem Ende anzuordnen. Die Bildverarbeitungseinrichtung ist vorzugsweise als externe Komponente vorgesehen, welche über Kabel mit der Strahlenquelle und dem Sensor verbunden ist. Es ist jedoch auch denkbar, die Bildverarbeitungseinrichtung direkt in das Endoskop oder in ein direkt an das Endoskop angesetztes Modul zu integrieren.

Die Messwerte, welche die Bildverarbeitungseinrichtung von dem Sensor bzw. dem Sensorelement für die einzelnen Bildpunkte erhält, umfassen vorzugsweise die Strahlungsintensität gemessen an den einzelnen Bildpunkten in Abhängigkeit von der Zeit. Hierbei muss keine kontinuierliche Erfassung und Auswertung der Intensität erfolgen, vielmehr kann es ausreichend sein, die Intensität der Strahlung an den einzelnen Bildpunkten zu vorbestimmten, aufeinander folgenden Zeitpunkten zu bestimmen, sodass der Phasenverlauf der Strahlung an den Bildpunkten bestimmt werden kann. In Kenntnis des Phasenverlaufes bzw. der Modulation an der Strahlenquelle können dann Phasendifferenzen bestimmt werden, auf deren Grundlagen dann die Signallaufzeiten an den einzelnen Bildpunkten bzw. insbesondere die Unterschiede der Laufzeiten zwischen den Signalen an den mehren Bildpunkten bestimmt werden, um die dreidimensionale Struktur des Objektes zu bestimmen.

Die Bildverarbeitungseinrichtung ist vorzugsweise zur Berechnung und Ausgabe der errechneten Entfernungswerte und ggf. der Ausgabe einer dreidimensionalen Darstellung des Objektes ausgebildet. Wahlweise können die Ergebnisse der Entfernungsmessung z.B. auf einem Monitor angezeigt und das Objekt in seiner dreidimensionalen Struktur für den Benutzer zusätzlich auf einem zweiten Monitor dargestellt werden.

Das endoskopische Instrument weist bevorzugt ein übliches Endoskop, vorzugsweise Videoendoskop zur Übertragung und Darstellung eines Bildes des zu untersuchenden Objektes auf. Dieses Endoskop ermöglicht es dem Benutzer, gleichzeitig während der Vermessung des zu untersuchenden Objekts dieses (vorzugsweise kontinuierlich) auf einem Monitor zu betrachten, was insbesondere bei medizinischen Anwendungen vorteilhaft sein kann.

Ferner weist das Endoskop vorzugsweise einen Lichtleiter auf, welcher zur Übertragung von Licht von einer proximalseitig angeordneten oder externen Lichtquelle zum distalen Ende ausgebildet ist, wobei das Licht zur Beleuchtung eines durch die Endoskopoptik zu beobachtenden Objektes vorgesehen ist. Des Weiteren kann im oder am distalen Ende des Endoskops eine entsprechende Lichtquelle angeordnet sein. Am distalen Ende des Endoskops tritt das Licht aus und beleuchtet den Bereich, der durch die Endoskopoptik bzw. das optische System beobachtbar ist. Besonders bevorzugt kann der Lichtleiter zusätzlich dazu genutzt werden, um die modulierte Strahlung vom proximalen zum distalen Ende des Endoskops zu übertragen, wobei die Strahlung dann am distalen Ende austritt. Diese Ausgestaltung ermöglicht es, die modulierte Strahlung vorzugsweise ohne zusätzliche Elemente durch das Endoskop bzw. durch den Endoskopschaft zu leiten, wenn die Strahlenquelle nicht direkt am distalen Ende des Endoskops angeordnet ist.

Nachfolgend wird die Erfindung beispielhaft anhand der beigefügten Figuren beschrieben. In diesen zeigt:
- Fig. 1: schematisch den Aufbau eines erfindungsgemäßen Instrumentes,
- Fig. 2: schematisch die Erfassung der Bewegung des erfindungsgemäßen Instrumentes bei einer ersten möglichen Anordnung der Beschleunigungsaufnehmer,
- Fig. 3: schematisch die Erfassung der Bewegung des medizinischen Instrumentes bei einer zweiten möglichen Anordnung der Beschleunigungsaufnehmer.
- Fig. 4: schematisch die Erfassung der Abwinkelbewegung eines abwinkelbaren medizinischen Instrumentes und
- Fig. 5: schematisch die Funktion des Time-Of-Flight-Kamerasystems.

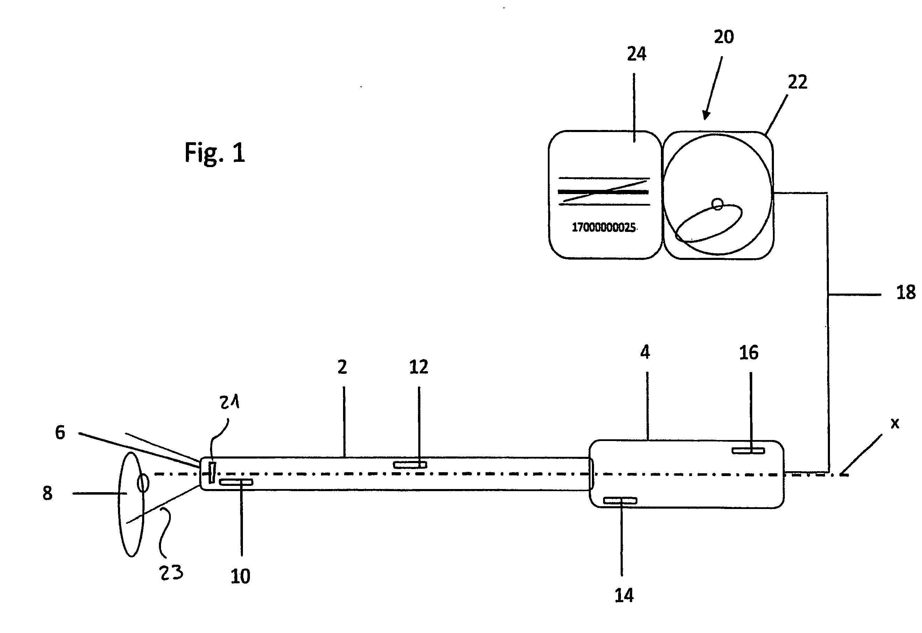

Das in Fig. 1 schematisch gezeigte medizinische Instrument in Form eines Endoskopes weist einen Schaft 2 mit einer proximalseitig angeordneten Handhabe 4 auf. Am distalen Ende weist der Schaft 2 ein Sichtfenster 6 auf, wobei hier schematisch die Sicht auf ein Objekt 8 gezeigt ist.

In dem Schaft 2 sind zwei Beschleunigungsaufnehmer 10 und 12 als Lagesensor angeordnet. Es handelt sich dabei vorzugsweise um Mehr-Achsen-Beschleunigungsaufnehmer, welche Bewegungen bzw. Beschleunigungen in mehreren, vorzugsweise zueinander orthogonalen Raumrichtungen erfassen können. Im gezeigten Beispiel sind vier Beschleunigungsaufnehmer 10, 12, 14, 16 vorgesehen, welche jeweils beabstandet zueinander in Richtung der Längsachse X sowie in diametraler Richtung quer zur Längsachse X angeordnet sind. Die Ausgangssignale der Beschleunigungsaufnehmer 10, 12, 14, 16 werden über geeignete Datenleitungen 18 einer Auswerteeinrichtung in Form eines Rechners zugeführt. Die Auswerteeinrichtung ist hier Teil einer Bildverarbeitungseinrichtung 20, welche nicht nur die Signale der Beschleunigungsaufnehmer 10 und 12, sondern gleichzeitig auch die von einem optischen Sensor in Form eines Video- bzw. Bildsensors 21 gelieferten Bildsignale verarbeitet. Die Bildverarbeitungseinrichtung 20 ist hier nicht in das Endoskop selber integriert, sondern bildet einen externen Bestandteil des endoskopisches Instrumentes. Die Bildverarbeitungseinrichtung 20 weist zwei Anzeigeelemente in Form von Bildschirmen 22 und 24 auf, wobei auf dem Bildschirm 22 in herkömmlicher Weise das aus dem Sichtfenster 6 von dem Bildsensor 21 aufgenommene Bild dargestellt und im Bildschirm 24 die Position des Endoskopes im Raum dargestellt wird.

Die gemeinsame Auswertung der Bildsignale und der Signale des Lagesensors in der Bildverarbeitungseinrichtung 20 ermöglichst es, Bilder in verschiedenen Blickrichtungen bzw. Lagen des Blickfeldes 23 aufzunehmen. Unter Berücksichtigung der jeweiligen Lage des optischen Sensors 21 und des Sichtfensters 6 und damit eher Blickrichtung im Raum kann so ein Gesamtbild aus den einzelnen Perspektiven des Objektes 8, idealerweise ein dreidimensionales Abbild erzeugt werden, sodass bei Betrachtung auf dem Bildschirm 22 und 24 das Objekt von verschiedenen Seiten betrachtet werden kann, ohne, das Instrument erneut bewegen zu müssen. Es kann somit ein virtuelles Modell des Objektes konstruiert werden.

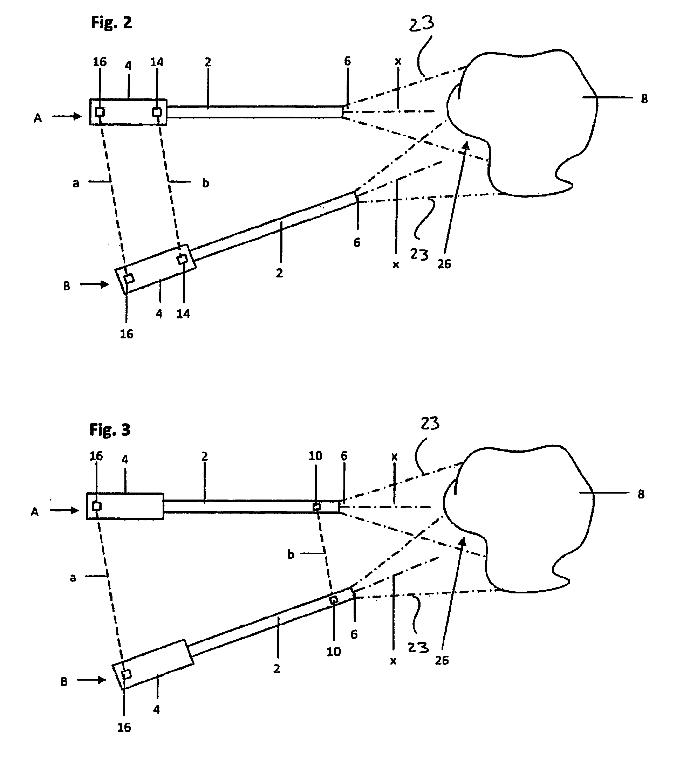

Anhand von Fig. 2 wird nun erläutert, wie mit Hilfe von zwei Beschleunigungsaufnehmern 14, 16 Lageänderungen des Endoskopes im Raum und insbesondere Änderungen der Blickrichtung erfasst werden können. In dem in Fig. 2 gezeigten Beispiel sind zwei Beschleunigungsaufnehmer 14 und 16 in axialer Richtung X beabstandet in der Handhabe 4 angeordnet. In Fig. 2 sind zwei Lagen des Instrumentes A und B dargestellt, welche unterschiedliche Blickrichtungen 23 zeigen. Es ist zu erkennen, dass in der Ausgangslage A beispielsweise der Bereich 26 des Objektes 8 nicht einsehbar ist. In der Lage B ist jedoch das Sichtfenster 6 auf den Bereich 26 gerichtet, so dass auch dieser einsehbar ist. So kann aus beiden Blickrichtungen gemeinsam ein dreidimensionales Gesamtbild des Objektes 8 von der Bildverarbeitungseinrichtung 20 zusammengesetzt werden. Die Lage A kann beispielsweise eine Lage sein, welche durch eine Kalibrierung der Bildverarbeitungseinrichtung 20 als Ausgangslage bekannt gemacht worden ist. Bei der Bewegung von der Lage A in die Lage B führen aufgrund der beabstandeten Anordnung die beiden Beschleunigungsaufnehmer 14 und 16 unterschiedliche Bewegungen aus, d. h. sie bewegen sich entlang unterschiedlicher Wege. Vereinfacht gezeigt ist hier der Weg a, welchen der Beschleunigungsaufnehmer 16 bei der Bewegung zurückzulegen hat, größer als der Weg b, welchen der Beschleunigungsaufnehmer 14 zurücklegt. Diese unterschiedlichen Bewegungen a und b führen zu unterschiedlichen Ausgangssignalen der Beschleunigungsaufnehmer 14 und 16, welche von der Bildverarbeitungseinrichtung 20 gebildeten Auswerteeinrichtung 20 erfasst werden. Aufgrund des definierten Abstandes zwischen den Beschleunigungsaufnehmern 14 und 16 in der Handhabe 4 und der bekannten Geometrie des Gesamtinstrumentes, insbesondere des Schaftes 2 kann die Bildverarbeitungseinrichtung 20 somit die Veränderung der Lage des gesamten Instrumentes im Raum bestimmen und insbesondere auch die Veränderung der Blickrichtung des Sichtfensters 6 aus den Ausgangssignalen der Beschleunigungsaufnehmer 14 und 16 und der bekannten Instrumentengeometrie bestimmen.

Fig. 3 zeigt die entsprechende Verlagerung des Instrumentes unter Verwendung von zwei Beschleunigungsaufnehmern 16 und 10, wobei der Beschleunigungsaufnehmer 16 am proximalen Ende der Handhabe 4 und der Beschleunigungsaufnehmer 10 am distalen Ende des Schaftes 2 angeordnet ist. Das heißt, hier ist der Abstand zwischen den beiden Beschleunigungsaufnehmern 10 und 16 in Richtung der Längsachse X des Instrumentes sehr lang. Aufgrund dieser Tatsache kommt es bei einer Schwenkbewegung, wie sie in Fig. 3 dargestellt ist, zu größeren Wegdifferenzen zwischen den Wegen a und b, welche die Beschleunigungsaufnehmer 10 und 16 bei der Schwenkbewegung zurücklegen. Dadurch lässt sich die Lageänderung des Instrumentes von der Position a zur Position b noch genauer erfassen. Ansonsten entspricht die Signalauswertung der Beschleunigungsaufnehmer 10 und 16 der vorangehend beschriebenen Auswertung der Ausgangssignale der Beschleunigungsaufnehmer 14 und 16 (hier Fig. 2).

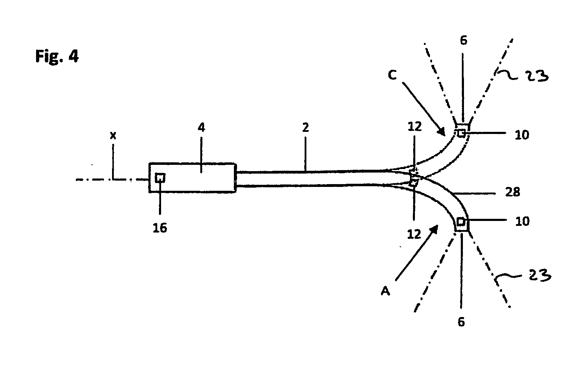

Fig. 4 zeigt eine weitere Variante, bei welcher der Schaft 2 an seinem distalen Ende einen biegbaren bzw. abwinkelbaren Bereich 28 aufweist. In Fig. 4 ist eine erste Position A des abwinkelbaren Bereiches 28 sowie eine zweite Position C (punktiert) dargestellt. Im hier gezeigten Beispiel sind drei Beschleunigungsaufnehmer 10, 12 und 16 vorgesehen. Der Beschleunigungsaufnehmer 16 liegt am proximalen Ende der Handhabe 4, während der Beschleunigungsaufnehmer 10 am distalen Ende des Schaftes 2 und der Beschleunigungsaufnehmer 12 beabstandet vom distalen Ende des Schaftes 2 im Schaft 2 angeordnet ist. Über die Ausgangssignale der Beschleunigungssensoren 10 und 12, welche vorzugsweise als Mehr-Achsen-Beschleunigungssensoren ausgebildet sind, kann deren Verlagerung im Raum von der Position A in die Position C von der Auswerteeinrichtung erfasst werden. Aus der bekannten Anordnung der Beschleunigungsaufnehmer 10 und 12 im Schaft 2 sowie der bekannten Geometrie des Schaftes 2 und dessen abwinkelbaren Abschnittes 28 kann die Bildverarbeitungseinrichtung 20 auf den Grad der Abwinkelung schließen und die Blickrichtung 23 des Sichtfensters 6 bzw. deren Veränderung bestimmen.

Es ist somit zu erkennen, dass durch die Anordnung mehrerer zueinander beabstandeter Beschleunigungsaufnehmer in dem endoskopisch Instrument Veränderungen dessen Lage im Raum bestimmt werden können. Insbesondere können auch Veränderungen der Blickrichtung 23 erfasst werden und die in den unterschiedlichen Blickrichtungen erzeugten Bilder von der Bildverarbeitungseinrichtung 20 zu einem dreidimensionalen Gesamtbild des Objektes 8 zusammengesetzt werden. Insbesondere ist auch die Erfassung von Änderungen der Blickrichtung möglich, wenn die Position der distalen Spitze des Instrumentes im Wesentlichen gleich bleibt und das Instrument lediglich um die distale Spitze des Instrumentes verschwenkt wird. Bei beabstandeter Anordnung der Beschleunigungsaufnehmer werden diese bei derartigen Schwenkbewegungen immer unterschiedlich lange Wege zurücklegen, so dass diese Schwenkbewegungen mit Drei-Achsen-Beschleunigungsaufnehmern erfasst werden können. Bewegungen entlang der Achsen der Beschleunigungsaufnehmer, d. h. Bewegungen in Richtung der Längsachse X bzw. in radialer Richtung zu dieser können direkt aus den Ausgangssignalen auch eines der Beschleunigungsaufnehmer von der Bildverarbeitungseinrichtung 20 bestimmt werden.

In der vorangehenden Beschreibung sind die Sensoren 10, 12, 14, 16 als Beschleunigungsaufnehmer beschrieben worden, welche Beschleunigungen entlang mehrerer Achsen erfassen. Es ist zu verstehen, dass auch einer oder mehrere dieser Sensoren 10, 12, 14, 16 anstatt als Mehr-Achsen-Beschleunigungssensor als Drehratensensor ausgeführt sein können. Insbesondere kann die Kombination eines Drehratenaufnehmers und eines Mehr-Achsen-Beschleunigungsaufnehmer Verwendung finden. So könnte beispielsweise der Aufnehmer 16 als Drehratenaufnehmer und der Aufnehmer 14 oder 10 als Mehrachsen-Beschleunigungsaufnehmer ausgebildet sein.

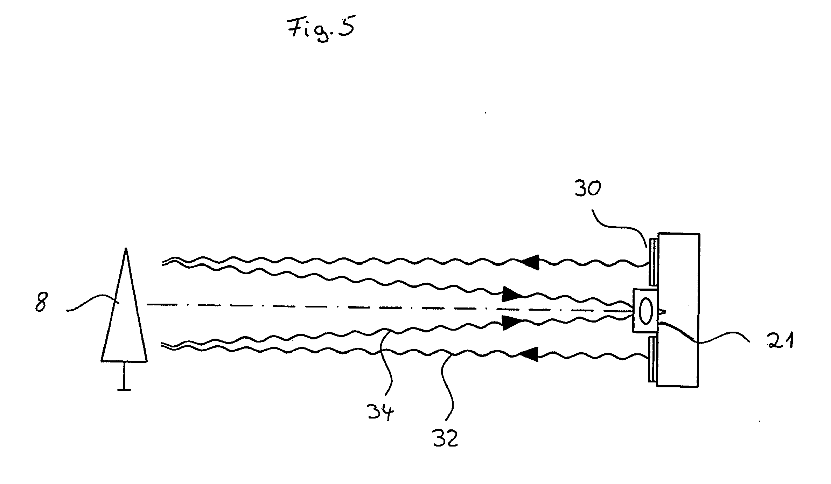

Wie anhand von Fig. 5 erläutert werden wird, ist der optische Sensor bzw. Bildsensor 21 Teile eines Time-Of-Flight-Kamerasystems. Fig. 5 zeigt schematisch die Funktion dieses Time-Of-Flight-Kamerasystems.

Das Instrument weist eine Beleuchtungseinrichtung 30 auf, welche seitlich des Bildsensors 21 im Wesentlichen in einer Ebene mit diesem platziert ist. Die Beleuchtungseinrichtung 30 ist so ausgebildet, dass sie moduliertes, d. h. insbesondere gepulstes Licht 30 (modulierte Strahlung) aussendet. Das gepulste Licht 32 liegt vorzugsweise im sichtbaren Bereich, sodass es über die üblichen optischen Elemente in dem Endoskop übertragbar ist.

Das gepulste Licht 32 trifft auf das Objekt 8 auf und wird von diesem als reflektiertes Licht 34 zurückgesendet und von dem Bildsensor 21 erfasst, der eine Vielzahl von Sensorelementen aufweist, von welchen jedes einem einzelnen Bildpunkt oder einer bestimmten Gruppe von Bildpunkten zugeordnet ist, sodass vorzugsweise im Wesentlichen das gesamte Objekt 8 durch einzelne Bildpunkte erfasst wird.

Aufgrund der dreidimensionalen Struktur des Objektes 8 sind die dem Sichtfenster 6 zugewandten Oberflächenbereiche des Objektes 8 unterschiedlich weit von dem Sichtfenster 6 und dem Bildsensor 21 beabstandet. Dies führt zu unterschiedlichen Laufzeiten des Lichtes von der Beleuchtungseinrichtung 30 zurück zu dem Bildsensor 21. Für die Bildpunkte, bei denen das Objekt 8 bzw. dessen Oberfläche weiter von dem Bildsensor 21 entfernt ist, ergeben sich längere Laufzeiten des Lichtes. Die Laufzeitunterschiede führen aufgrund der Modulation des ausgesendeten Lichtes 32 zu Phasendifferenzen, die von der Bildverarbeitungseinrichtung 20 erfasst und ausgewertet werden, um die Laufzeit und damit die Entfernung der Bildpunkte zu bestimmen. Hieraus wiederum kann die Bildverarbeitungseinrichtung 20 dann die dreidimensionale Struktur des Objektes 2 ableiten. Die Funktionsweise einer solchen TOF-Kamera ist beispielsweise aus DE 4 440 613 C1, EP 1 952 752 oder EP 1 622 200 A1 bekannt.

Dadurch, dass die Bildverarbeitungseinrichtung 20 sowohl die von dem Lagesensor, welcher durch die Beschleunigungsaufnehmer 10, 12, 14, 16 gebildet wird, erzeugten Positionssignale und Informationen über die dreidimensionale Struktur des Objektes 8 von dem TOF-Kamerasystem gemeinsam auswertet, kann eine optimierte Objektrekonstruktion in der Bildverarbeitungseinrichtung 20 erreicht werden. Es werden dreidimensionale Strukturen sowohl durch Berücksichtigung unterschiedlicher Blickrichtungen, als auch durch die Laufzeitsignale ermittelt. Durch die Berücksichtigung der unterschiedlichen Blickrichtungen können dabei, wie anhand von Fig. 2 und 3 erläutert, auch Bereiche des Objektes erkannt und dargestellt werden, welche mit nur einer Blickrichtung 23 nicht erkennbar wären, beispielsweise weil sie hinter anderen Objektbereichen gelegen sind.

### Bezugszeichenliste

- 2: - Schaft
- 4: - Handhabe
- 6: - Sichtfenster
- 8: - Objekt
- 10, 12, 14, 16: - Beschleunigungsaufnehmer
- 18: - Datenleitung
- 20: - Bildverarbeitungseinrichtung
- 23: - Blick, Blickrichtung
- 22, 24: - Bildschirme
- 26: - Bereich des Objekts
- 28: - abwinkelbarer Bereich
- 30: - Beleuchtungseinrichtung
- 32: - Licht
- 34: - reflektiertes Licht

- X: - Instrumentenlängsachse
- A, B, C: - Positionen des Instrumentes
- a, b: - Bewegungen der Beschleunigungsaufnehmer

## Patentansprüche

1. Endoskopisches Instrument mit zumindest einem optischen Sensor (21) und zumindest einem Lagesensor (10, 12, 14, 16), welcher geeignet ist, eine Lageänderung des Blickfeldes (23) des optischen Sensor (21) im Raum zu erfassen,
sowie einer Bildverarbeitungseinrichtung (20), welche derart ausgestaltet ist, dass sie Ausgangssignale des optischen Sensors und Ausgangssignale des Lagesensors (10, 12, 14, 16) gemeinsam derart verarbeitet, dass von dem optischen Sensor (21) in verschiedenen Lagen des Blickfeldes (23) erfasste Bildinformationen unter Berücksichtigung der Lage des Blickfeldes 23 zu einem Gesamtbild eines betrachteten Objektes (8) zusammengesetzt werden, **dadurch gekennzeichnet, dass** der optische Sensor (21) Teil eines Time-Of-Flight-Kamerasystems ist und das endoskopische Instrument eine Strahlquelle (30) aufweist, welche eine modulierte Strahlung (32) in Richtung des Blickfeldes (23) des optischen Sensors (21) aussendet,
der optische Sensor (21) mit mehreren Sensorelementen zum Erfassen der von einem Objekt (8) reflektierten Strahlung (34) an verschiedenen Bildpunkten versehen ist und
die Bildverarbeitungseinrichtung (20) derart ausgebildet ist, dass sie von den einzelnen Sensorelementen Messwerte der erfassten reflektierten Strahlung (34) erhält und basierend auf diesen Messwerten an den einzelnen Bildpunkten Phasendifferenzen bestimmt und auf deren Grundlage die Entfernung der Bildpunkte von dem optischen Sensor (21) und so ein dreidimensionales Abbild des Objektes (8) errechnet.

2. Endoskopisches Instrument nach Anspruch 1, **dadurch gekennzeichnet, dass** der optische Sensor (21) ein Videosensor ist, welcher vorzugsweise am distalen Ende des Endoskops angeordnet ist.

3. Endoskopisches Instrument nach einem der Ansprüche 1 bis 2, **dadurch gekennzeichnet, dass** der Lagesensor zumindest zwei Bewegungsaufnehmer (10, 12, 14, 16) und eine Auswerteeinrichtung aufweist, wobei Ausgangssignale der Bewegungsaufnehmer (10, 12, 14, 16) der Auswerteeinrichtung zugeführt werden und die Auswerteeinrichtung derart ausgebildet ist, dass sie auf Grundlage der Ausgangssignale der Bewegungsaufnehmer (10, 12, 14, 16) Veränderungen der Lage des Blickfeldes (23) im Raum erfasst.

4. Endoskopisches Instrument nach Anspruch 3, **dadurch gekennzeichnet, dass** die zumindest zwei Bewegungsaufnehmer (10, 12, 14, 16) voneinander beabstandet angeordnet sind.

5. Endoskopisches Instrument nach Anspruch 3 oder 4, **dadurch gekennzeichnet, dass** zumindest einer der Bewegungsaufnehmer ein Beschleunigungs- oder Drehratenaufnehmer oder ein Mehrachsen-Beschleunigungsaufnehmer (10, 12, 14, 16) ist.

6. Endoskopisches Instrument nach einem der Ansprüche 3 bis 5, **dadurch gekennzeichnet, dass** zumindest ein Bewegungsaufnehmer (10, 12, 14, 16) in einer distalseitigen Hälfte und/oder zumindest ein Bewegungsaufnehmer (14, 16) in einer proximalseitigen Hälfte des Instrumentes angeordnet ist.

7. Endoskopisches Instrument nach einem der Ansprüche 3 bis 6, **gekennzeichnet durch** einen Schaft mit zumindest einem abwinkelbaren Schaftabschnitt (28)**,** wobei zumindest ein Bewegungsaufnehmer (10) in dem abwinkelbaren Schaftabschnitt (28) angeordnet ist.

8. Endoskopisches Instrument nach einem der vorhergehenden Ansprüche, **dadurch gekennzeichnet, dass** die Strahlquelle (30) moduliertes Licht vorzugsweise im sichtbaren Bereich aussendet.

9. Endoskopisches Instrument nach einem der vorhergehenden Ansprüche, **dadurch gekennzeichnet, dass** die Strahlquelle (30) zum Aussenden von gepulstem Licht als modulierte Strahlung ausgebildet ist.

10. Endoskopisches Instrument nach einem der vorhergehenden Ansprüche, **dadurch gekennzeichnet, dass** ein erster optischer Sensor (21) zum Erfassen der von einem Objekt reflektierten Strahlung und ein Videosensor oder eine Optik mit einem Okular zur direkten Betrachtung des Objektes vorhanden sind.

11. Endoskopisches Instrument nach Anspruch 10, **dadurch gekennzeichnet, dass** der erste optische Sensor (21) und der Videosensor oder das Okular über einen gemeinsamen optischen Pfad mit einem Sichtfenster am distalen Ende des endoskopischen Instrumentes verbunden sind.

## Claims

1. An endoscopic instrument with at least one optical sensor (2) and with at least one position sensor (10, 12, 14, 16) which is suitable for detecting a position change of the viewing field (23) of the optical sensor (21) in space,
as well as with a picture processing device (20) which is designed in a manner such that it commonly processes output signals of the optical sensor and output signals of the position sensor (10, 12, 14, 16) in a manner such that picture information which is detected by the optical sensor (21) in different positions of the viewing field (23) are put together into a total picture of a considered object (8) whilst taking the position of the viewing field (23) into account, **characterised in that** the optical sensor (21) is part of a time-of-flight camera system and the endoscopic instrument comprises a beam source (30) which emits a modulated radiation (32) in the direction of the viewing field (23) of the optical sensor (21),
the optical sensor (21) is provided with several sensor elements for detecting the radiation (34) reflected by an object (8) at different picture points and
the picture processing device (20) is designed in a manner such that it receives readings of the detected reflected radiation (34) from the individual sensor elements, and determines phase differences on the basis of these readings at the individual picture points, and computes the distance of the picture points to the optical sensor (2) and thus a three-dimensional image of the object (8) on the basis of said phase differences.

2. An endoscopic instrument according to claim 1, **characterised in that** the optical sensor (21) is a video sensor which is preferably arranged at the distal end of the endoscope.

3. An endoscopic instrument according to one of the claims 1 to 2, **characterised in that** the position sensor comprises at least two movement sensors (10, 12, 14, 16) and an evaluation device, wherein output signals of the movement sensors (10, 12, 14, 16) are led to the evaluation device and the evaluation device is designed such that it detects changes of the position of the viewing field (23) in space, on the basis of the output signals of the movement sensors (10, 12, 14, 16).

4. An endoscopic instrument according to claim 3, **characterised in that** the at least two movement sensors (10, 12, 14, 16) are arranged distanced to one another.

5. An endoscopic instrument according to claims 3 or 4, **characterised in that** at least one of the movement sensors is an acceleration sensor or rotation rate sensor or a multi-axis acceleration sensor (10, 12, 14, 16).

6. An endoscopic instrument according to one of the claims 3 to 5, **characterised in that** at least one movement sensor (10, 12, 14, 16) is arranged in a distal-side half and/or at least one movement sensor (14, 16) is arranged in a proximal-side half of the instrument.

7. An endoscopic instrument according to one of the claims 3 to 6, **characterised by** a shank with at least one shank section (28) which can be angled, wherein at least one movement sensor (10) is arranged in the shank section (28) which can be angled.

8. An endoscopic instrument according to one of the preceding claims, **characterised in that** the beam source (30) emits modulated light, preferably in the visible range.

9. An endoscopic instrument according to one of the preceding claims, **characterised in that** the beam source (30) is designed for emitting pulsed light as modulated radiation.

10. An endoscopic instrument according to one of the preceding claims, **characterised in that** a first optical sensor (21) for detecting the radiation reflected by an object, and a video sensor or optics with an ocular for the direct observation of the object are present.

11. An endoscopic instrument according to claims 10, **characterised in that** the first optical sensor (21) and the video sensor or the ocular are connected via a common optical path to a viewing window at the distal end of the endoscopic instrument.

## Revendications

1. Instrument endoscopique comportant au moins un capteur optique (21) et au moins un capteur de position (10, 12, 14, 16) qui est approprié pour détecter une modification de la position du champ de vision (23) du capteur optique (21) dans l'espace,
ainsi qu'un dispositif de traitement d'image (20) qui est constitué de telle sorte qu'il traite ensemble des signaux de sortie du capteur optique et des signaux de sortie du capteur de position (10, 12, 14, 16) de telle sorte que des données d'image détectées par le capteur optique (21) dans différentes positions du champ de vision (23) sont rassemblées en une image complète d'un objet observé (8) en prenant en compte la position du champ de vision (23), **caractérisé en ce que** le capteur optique (21) fait partie d'un système de caméras à temps de vol et que l'instrument endoscopique présente une source de rayonnement (30) qui émet un rayonnement modulé (32) en direction du champ de vision (23) du capteur optique (21),
le capteur optique (21) est muni de plusieurs éléments capteurs servant à détecter le rayonnement (34) réfléchi par l'objet (8) en différents points d'image, et
le dispositif de traitement d'image (20) est conçu de telle sorte qu'il reçoit en provenance des différents éléments capteurs des valeurs de mesure du rayonnement réfléchi détecté (34) et qu'en se basant sur ces valeurs de mesure, il détermine des différences de phase entre les différents points d'image et, d'après ces dernières, calcule la distance entre les points d'image et le capteur optique (21), et ainsi une représentation tridimensionnelle de l'objet (8).

2. Instrument endoscopique selon la revendication 1, **caractérisé en ce que** le capteur optique (21) est un capteur vidéo qui est disposé de préférence à l'extrémité distale de l'endoscope.

3. Instrument endoscopique selon l'une des revendications 1 à 2, **caractérisé en ce que** le capteur de position présente au moins deux capteurs de mouvement (10, 12, 14, 16) et un dispositif d'analyse, les signaux de sortie des capteurs de mouvement (10, 12, 14, 16) étant transmis au dispositif d'analyse et le dispositif d'analyse étant conçu de manière à détecter des changements de position du champ de vision (23) sur la base des signaux de sortie des capteurs de mouvement (10, 12, 14, 16).

4. Instrument endoscopique selon la revendication 3, **caractérisé en ce que** lesdits deux capteurs de mouvement (10, 12, 14, 16), au moins au nombre de deux, sont disposés à distance l'un de l'autre.

5. Instrument endoscopique selon la revendication 3 ou 4, **caractérisé en ce qu'**au moins l'un des capteurs de mouvement est un capteur d'accélération ou de taux de rotation ou un capteur d'accélération multiaxe (10, 12, 14, 16).

6. Instrument endoscopique selon l'une des revendications 3 à 5, **caractérisé en ce qu'**au moins un capteur de mouvement (10, 12, 14, 16) est disposé dans une moitié distale et/ou au moins un capteur de mouvement (14, 16) disposé dans une moitié proximale de l'instrument.

7. Instrument endoscopique selon l'une des revendications 3 à 6, **caractérisé par** une tige possédant au moins un segment de tige pouvant être coudé (28), au moins un capteur de mouvement (10) étant disposé dans le segment de tige pouvant être coudé (28).

8. Instrument endoscopique selon l'une des revendications précédentes, **caractérisé en ce que** la source de rayonnement (30) émet une lumière modulée, de préférence dans le domaine visible.

9. Instrument endoscopique selon l'une des revendications précédentes, **caractérisé en ce que** la source de rayonnement (30) est conçue pour émettre une lumière pulsée en tant que rayonnement modulé.

10. Instrument endoscopique selon l'une des revendications précédentes, **caractérisé en ce qu'**il comporte un premier capteur optique (21) destiné à détecter le rayonnement réfléchi par un objet et un capteur vidéo ou une optique comportant un oculaire pour l'observation directe de l'objet.

11. Instrument endoscopique selon la revendication 10, **caractérisé en ce que** le premier capteur optique (21) et le capteur vidéo ou l'oculaire sont reliés par un trajet optique commun à une fenêtre située à l'extrémité distale de l'instrument endoscopique.
